# EUROPEAN PATENT SPECIFICATION

(11) **EP 2 011 677 B1**
(45) Date of publication and mention of the grant of the patent: **30.12.2009**
(21) Application number: 07425403.8
(22) Date of filing: 02.07.2007
(51) Int. Cl.: B60H 3/00

(54) **Device and method for perfuming the compartment of a vehicle**
Vorrichtung und Verfahren zum Beduften eines Fahrzeuginnenraums
Appareil et procédé pour parfumer l'habitacle d'une voiture

(43) Date of publication of application: 07.01.2009
(73) Proprietor: Magneti Marelli S.p.A., 20011 Corbetta (MI) (IT)
(72) Inventor: Zompi, Roberto c/o Magneti Marelli Sistemi Elettronici S.p.A., 10078 Venaria Reale (Torino) (IT)
(74) Representative: Marchitelli, Mauro

(56) References cited:
- EP-A- 1 439 083
- US-A1- 2003 206 834
- US-B1- 6 357 726

## Description

The present invention relates to devices for perfuming the compartment of a vehicle. Examples of these devices are disclosed in EP 1 439 083 A and US 2003/0206834 which represents the closest prior art. Devices of this type which foresee a drive electronic unit which allows to select a preferred fragrance amongst at least two available fragrances and to adjust the intensity with which the selected fragrance is introduced in the compartment of the vehicle have already been proposed (FR-A-2 867 424).

The object of the present invention is to carry out a device of the type above specified which presents an extremely simple structure and with a comfortable and easy use and which, at the same time, allows the user to have at his disposal additional functions in predisposing the perfuming of the compartment.

In view of attaining such purpose, the subject of the invention is a device for perfuming the compartment of a vehicle having the features of claim 1.

Thanks to the aforesaid features, the device according to the invention allows the user not only to select a fragrance amongst different fragrances arranged within the reservoirs of the device, but also and above all to mix together different fragrances, according to adjustable proportions, for obtaining the possibility of introducing within the compartment of the vehicle a fragrance which can be selected from a number of different fragrances higher than the number of base fragrances which fill the reservoirs of the device.

Of course, the system can be arranged such that the user is completely free to compose" the fragrance of his choice, by varying as much as he wishes the quantity of each of the selected components, or it can be foreseen that the electronic control system is programmed for allowing the selection amongst a series of predetermined mixtures.

In a preferred embodiment, the connector element carried by the device is a USB-type plug intended for being received in a complementary seat arranged on a control panel of the dashboard of the vehicle. On this control panel, adjusting means are further foreseen, for example in form of keys or knobs, for allowing the user to carry out the selection of the components of the mixture and their quantity, as well as a display panel, if necessary.

The mixture delivery of the selected fragrances in the environment is preferably carried out through a diffusing plate, partly dipped within the mixing chamber and partly exposed to the outside and excited (for example through ultrasounds or through Joule effect heating) for the purpose of diffusing the mixture of selected fragrances within the compartment of the vehicle.

Further features and advantages of the present invention will result from the following description with reference to the enclosed drawings, given by mere way of not limiting example, in which:

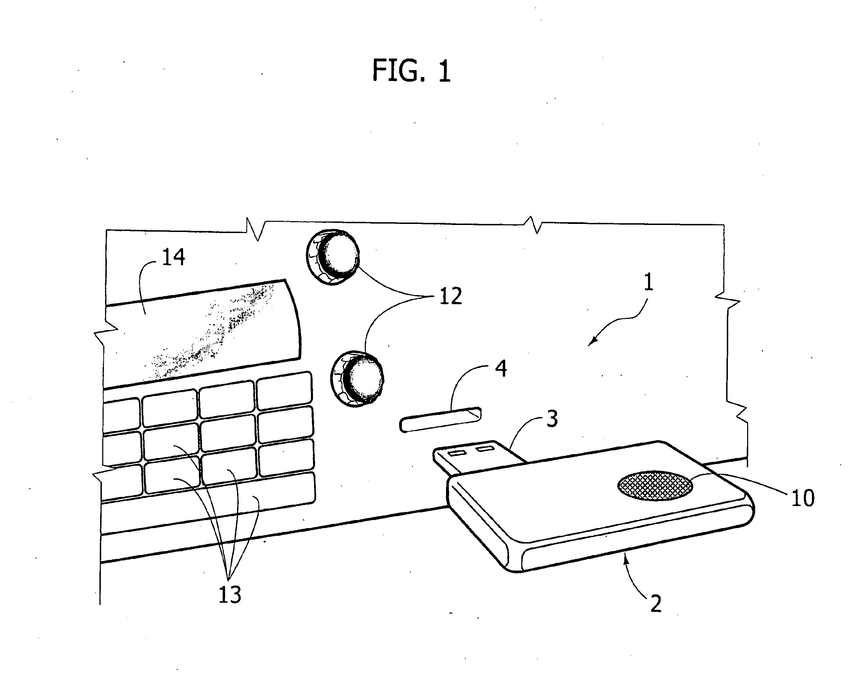

- figure 1 is a diagrammatic perspective view of the device according to the invention, and
- figure 2 is a block diagram which shows the device structure of figure 1.

With reference to the drawings, numeral 1 generally shows a device for perfuming the compartment of a vehicle, including a body 2 from which a USB-type connector 3 is protruding, intended for being received within a complementary connector such that to allow the connection of an electronic control unit 5 arranged within the device 2 with the board electronic unit 6 of the vehicle.

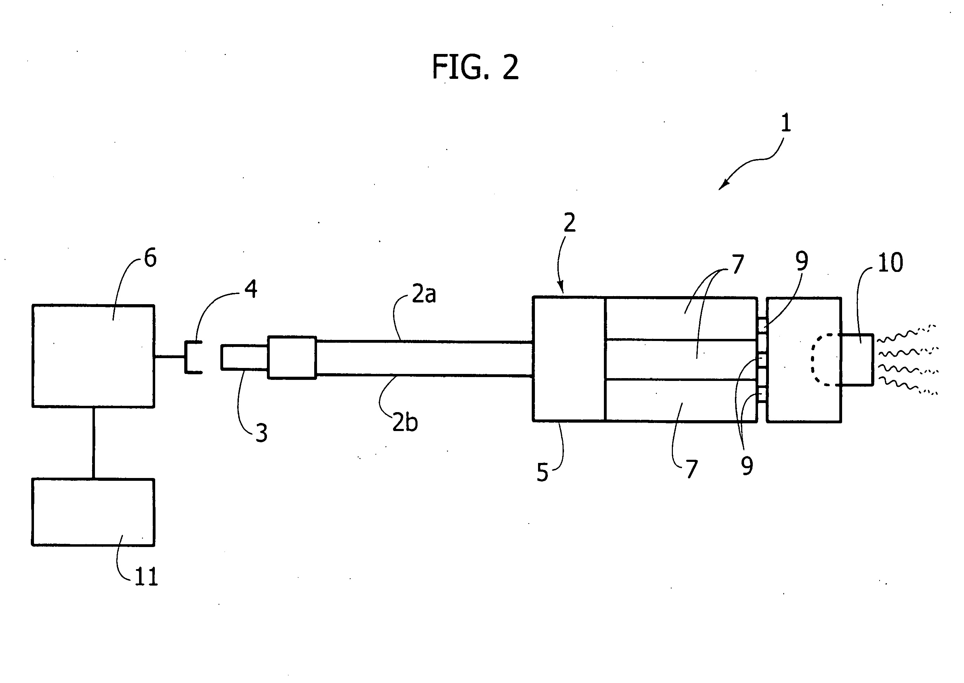

With reference to figure 2, the body 2 of the device 1 further includes a plurality of reservoirs 7 filled with different fragrances at the liquid state, each of which is able to deliver a jet of microdrops of the fragrance contained therein within a mixing chamber 8, through a delivery solenoid valve 9, each reservoir 7 being equipped with a respective solenoid valve 9. The structure and the conformation of the solenoid valve 9 is not shown in detail herein, as it can be carried out in any known way, for example analogously with the technology foreseen in ink-jet printer cartridges.

To the mixing chamber 8 a plate 10 for diffusing in the environment the mixture of selected fragrances is associated. The plate 10 shows a portion dipped in the mixing chamber 8 and a part 10a exposed to the outside, for the diffusion of the fragrance in the environment.

The functioning of the adjusting solenoid valves 9 is controlled by the electronic control unit 5 based on signals sent from the board electronic unit 6 of the vehicle, through the connection 3, 4, a power line 2a and a control line 2b. The on-board electronic unit 6 is associated with an adjusting and, if necessary, display unit 11 arranged on the vehicle dashboard and having adjusting elements, for example in form of knobs 12 and/or keys 13 which allow the user to select the fragrances to be mixed and the quantity of each of them, possibly with the help of a display 14. Based on the commands imparted by the user to the unit 11, the unit 6 sends instructions to the electronic unit 5 of the device 1, so as to consequently regulate the adjusting solenoid valves 9. The diffusion plate 10 is excited, for example through ultrasounds or through Joule effect heating, so as to deliver in the environment the mixture of selected fragrances.

Naturally, construction details could widely vary with respect to what has been described and shown by mere way of example, without leaving for this reason the scope of the attached claims.

## Claims

1. Device adapted to be arranged on a vehicle for perfuming the compartment of the vehicle, **characterized in that** it includes:
- a body (2), including a plurality of reservoirs (7) filled with different fragrances at the liquid state,
- a delivery solenoid valve (9) of the type providing a jet of microdrops, associated with each reservoir (9),
- a mixing chamber (8) of jets exiting from the solenoid valves (9) associated with the aforesaid reservoirs (7),
- an environment diffusion element (10) of the mixed fragrance, at least partly dipped within the mixing chamber (9) and having a portion exposed to the outside,
- an electronic control unit (5) carried by the aforesaid body (2), for controlling the delivery solenoid valves (9) of the fragrances,
- a connector element (3) carried by the aforesaid body, and a complementary connector element (4) for receiving said connector element and adapted to be arranged on the vehicle, for the connection of said electronic control unit (5) of the perfuming device with an on-board electronic unit (6) of the vehicle, and
- adjusting means (11) adapted to be arranged on the vehicle and adapted to be connected with said control unit (5) of the device via the on-board control unit (6) of the vehicle, for adjusting the selection of the fragrances to be mixed and their quantity.

2. Device according to claim 1, **characterized in that** said diffusion organ (10) consists of a plate which is excited through ultrasounds or by Joule effect heating.

3. Device according to claim 1, **characterized in that** said connector element (3) consists of a connector of the USB type.

4. Vehicle comprising a device according to claim 1, wherein said complementary connector element (4) is arranged on the vehicle dashboard.

## Patentansprüche

1. Vorrichtung, geeignet zur Anordnung auf einem Fahrzeug zum Beduften des Innenraums des Fahrzeugs, **dadurch gekennzeichnet, dass** sie umfasst:
- einen Körper (2) mit einer Vielzahl von Reservoirs (7), die mit verschiedenen Düften in flüssigem Zustand gefüllt sind,
- ein Zufuhrmagnetventil (9) zur Bereitstellung eines Strahls von Mikrotropfen, welches mit jedem Reservoir (9) verbunden ist,
- eine Mischungskammer (8) für Strahle, die aus den Magnetventilen (9) austreten, die mit den Reservoirs (7) verbunden sind,
- ein Umwelt-Diffusionselement (10) des gemischten Dufts, welches zumindest teilweise in die Mischungskammer (9) eintaucht, und einen Abschnitt aufweist, welcher der Außenseite ausgesetzt ist,
- eine elektronische Steuereinheit (5), die durch den Körper (2) getragen wird, zum Steuern der Zufuhrmagnetventile (9) der Düfte,
- ein Verbinderelement (3), welches durch den Körper getragen wird, und ein entgegengesetztes Verbinderelement (4) zur Aufnahme des Verbinderelements, angepasst zur Anordnung auf dem Fahrzeug zur Verbindung der elektronischen Steuereinheit (5) der Beduftungsvorrichtung mit einer Fahrzeug-eigenen elektronischen Einheit (6) des Fahrzeugs, und
- Einstellmittel (11), die zur Anordnung auf dem Fahrzeug angepasst sind, und die zur Verbindung mit der Steuereinheit (5) der Vorrichtung über die Fahrzeug-eigene Steuereinheit (6) des Fahrzeugs zum Einstellen der Auswahl von zu mischenden Düften und ihrer Quantität angepasst sind.

2. Vorrichtung nach Anspruch 1, **dadurch gekennzeichnet, dass** das Diffusionsorgan (10) aus einer Platte besteht, welche durch Ultraschall oder durch eine Joule-Effekt-Heizung angeregt wird.

3. Vorrichtung nach Anspruch 1, **dadurch gekennzeichnet, dass** das Verbinderelement (3) aus einem Verbinder der USB-Art besteht.

4. Fahrzeug umfassend eine Vorrichtung nach Anspruch 1, wobei das entgegengesetzte Verbinderelement (4) auf der Fahrzeuginstrumententafel angeordnet ist.

## Revendications

1. Dispositif apte à être disposé sur un véhicule pour parfumer l'habitacle du véhicule, **caractérisé en ce qu'**il comprend :
- un corps (2), comprenant une pluralité de réservoirs (7) remplis de différentes senteurs à l'état liquide,
- une électrovanne de délivrance (9) du type produisant un jet de microgouttes, associée à chaque réservoir (9),
- une chambre de mélange (8) des jets sortant des électrovannes (9) associée aux réservoirs (7) précités,
- un élément de diffusion dans l'environnement (10) de la senteur mélangée, trempant au moins partiellement à l'intérieur de la chambre de mélange (9) et ayant une partie exposée à l'extérieur,
- une unité de contrôle électronique (5) portée par le corps (2) précité, pour contrôler les électrovannes de délivrance (9) des senteurs,
- un élément de connecteur (3) porté par le corps précité, et un élément de connecteur complémentaire (4) pour recevoir ledit élément de connecteur et apte à être disposé sur le véhicule, pour la connexion de ladite unité de contrôle électronique (5) du dispositif de parfumage avec une unité électronique embarquée (6) du véhicule, et
- des moyens d'ajustement (11) aptes à être disposés sur le véhicule et aptes à être connectés à ladite unité de contrôle (5) du dispositif via l'unité de contrôle embarquée (6) du véhicule, pour ajuster la sélection des senteurs à mélanger et leur quantité.

2. Dispositif selon la revendication 1, **caractérisé en ce que** ledit organe de diffusion (10) est constitué d'une plaque qui est excitée par des ultrasons ou par chauffage par effet Joule.

3. Dispositif selon la revendication 1, **caractérisé en ce que** ledit élément de connecteur (3) consiste en un connecteur du type USB.

4. Véhicule comprenant un dispositif selon la revendication 1, dans lequel ledit élément de connecteur complémentaire (4) est disposé sur le tableau de bord du véhicule.
